# EUROPEAN PATENT APPLICATION

(11) **EP 4 729 511 A1**
(43) Date of publication of application: **22.04.2026**
(21) Application number: 24823390.0
(22) Date of filing: 12.06.2024
(51) Int. Cl.: C07D 233/84, A61K 31/4178, A61P 43/00

(54) **COMPOUND OR SALT THEREOF AND METHOD FOR PRODUCING SAME, COMPOSITION, HYDROGEN PEROXIDE REMOVER, AND CYTOTOXICITY INHIBITOR**

(30) Priority: 15.06.2023 JP 2023098209
(71) Applicant: Nagase & Co., Ltd., Osaka-shi, Osaka 550-8668 (JP)
(72) Inventor: MATSUMOTO,Jun, Kobe-shi, Hyogo 651-2241 (JP); KOSAKA,Kunio, Kobe-shi, Hyogo 651-2241 (JP)
(74) Representative: dompatent
(86) International application number: PCT/JP2024/021265
(87) International publication number: WO 2024/257775

(57) **Abstract**

Provided are a novel ergothioneine derivative and a method for producing the same; a composition containing the derivative; a hydrogen peroxide remover, and a cytotoxicity inhibitor. A compound represented by the following formula (1) or a salt thereof.

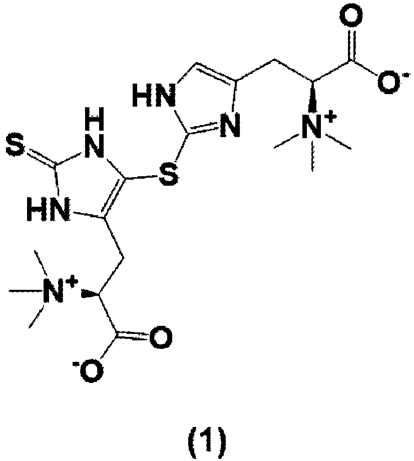

## Description

### TECHNICAL FIELD

The present invention relates to a novel ergothioneine derivative and a method for producing the same, a composition, a hydrogen peroxide remover, and a cytotoxicity inhibitor comprising said derivative, and the like.

### BACKGROUND ART

L-ergothioneine (also referred to as "EGT" in the present description) is a kind of sulfur-containing amino acid and is known to have various physiological activities.

Meanwhile, as compositions having hydrogen peroxide removal effects, antioxidant functional water containing fine bubbles and the like are known to date. (for example, see Patent Document 1). Also, as compositions having cytotoxicity inhibiting effects, a intracellular thioredoxin system activator containing hinokitiol and the like are known to date (for example, see Patent Document 2).

However, there are wide variety of uses and targets etc. requiring hydrogen peroxide removal effects or cytotoxicity inhibiting effects, and the development of new hydrogen peroxide removers or cytotoxicity inhibitors is required.

### CITATION LIST

### PATENT DOCUMENT

Patent Document 1: JP2008-156320A
Patent Document 2: JP2020-103100A

### SUMMARY OF THE INVENTION

### PROBLEMS TO BE SOLVED BY THE INVENTION

In light of these circumstances, an object of the present invention is to provide a novel ergothioneine derivative and a method for producing the same, a composition, a hydrogen peroxide remover, and a cytotoxicity inhibitor comprising said derivative, and the like.

### MEANS TO SOLVE PROBLEMS

The present inventors have earnestly studied to solve the above problems, and as a result, they succeeded in producing the following novel ergothioneine derivative (a compound represented by the following formula 1 or a salt thereof.), found that the above problems can be solved by the above compound, and completed the present invention.

Namely, the present invention provides the following compound or a salt thereof (hereinafter also referred to as "compound" or "compound etc.").

### Item 1.

A compound represented by the following formula (1) or a salt thereof

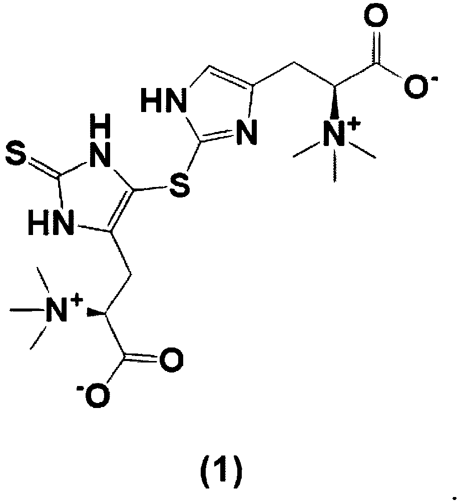

Also, the present invention provides the following composition.

### Item 2.

A composition comprising the compound according to Item 1 or a salt thereof.

Also, the present invention provides the following hydrogen peroxide removers.

### Item 3.

A hydrogen peroxide remover comprising the compound according to Item 1 or a salt thereof.

### Item 4.

The hydrogen peroxide remover according to Item 3, wherein a concentration of the above compound or a salt thereof is used at a concentration of 0.04 µM or more.

Also, the present invention provides the following cytotoxicity inhibitor.

### Item 5.

A cytotoxicity inhibitor comprising the compound according to Item 1 or a salt thereof, which is used in the presence of hydrogen peroxide.

Also, the present invention relates to the following method for producing.

### Item 6.

A method for producing a compound represented by the following formula (1) or a salt thereof, which comprises a step (1) for heat-treating an aqueous solution of L-ergothioneine at 60°C or more for 24 hours or more

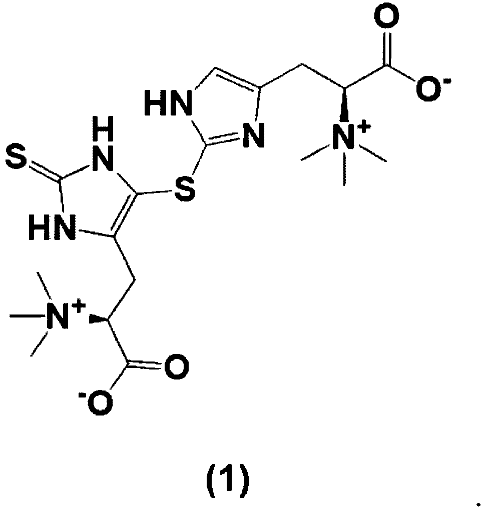

### EFFECT OF INVENTION

The compound or a salt thereof of the present invention and a composition comprising the same can be appropriately used as, for example a hydrogen peroxide remover, a cytotoxicity inhibitor, or the like.

Also, the hydrogen peroxide remover of the present invention can achieve, for example, an effect for appropriately removing hydrogen peroxide.

Also, the cytotoxicity inhibitor of the present invention can achieve, for example, an effect for reducing the cytotoxicity caused by hydrogen peroxide in a medium.

Also, the above compound or a salt thereof and the like can be easily obtained by using the method for producing the compound of the present invention.

### BRIEF DESCRIPTION OF DRAWINGS

[Figure 1] Figure 1 is a graph showing the HPLC analysis results after 96 hours heat-treatment in Example 1.
[Figure 2] Figure 2 is a graph showing the measurement results of liquid chromatography-high resolution mass spectrometry (LC-HRMS) in Example 1.
[Figure 3] Figure 3 is a graph showing the evaluation results of residual hydrogen peroxide amount in Example 2.
[Figure 4] Figure 4 is a table showing the evaluation results of residual hydrogen peroxide amount in Example 2.
[Figure 5] Figure 5 is a photo diagram showing the evaluation results of hydrogen peroxide cytotoxicity against HEK293 cells in Example 3.
[Figure 6] Figure 6 is a graph showing the evaluation results of hydrogen peroxide cytotoxicity against HEK293 cells in Example 3.
[Figure 7] Figure 7 is a graph showing the evaluation results of hydrogen peroxide cytotoxicity against HEK293 cells in Example 3.
[Figure 8] Figure 8 is a photo diagram showing the evaluation results of hydrogen peroxide cytotoxicity against HEK293 cells in Example 3.

### MODE FOR CARRYING OUT THE INVENTION

Hereinafter, embodiments of the present invention are described in detail.

### [Compound or salt thereof]

The compound or a salt thereof of the present invention (hereinafter also referred to as "Compound (1)") is represented by the following formula 1

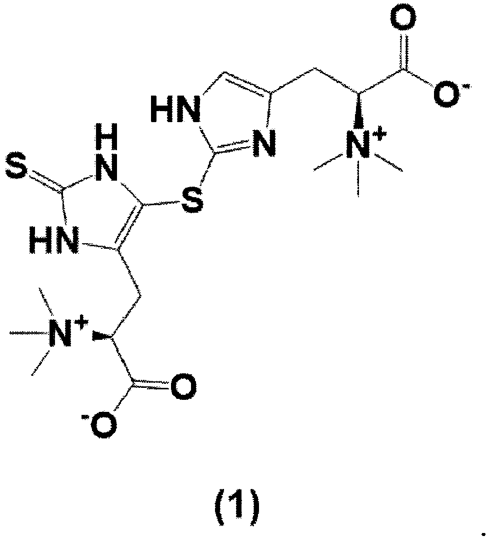

The above compound is a kind of oxidized dimer of L-ergothioneine.

The salt of the above compound may be an intramolecular salt in these structures, and another counter cation and/or counter anion may be included.

The salt of the above compound may be, for example, a pharmacologically or physiologically acceptable salt. The above salt is not specifically limited, and specific examples thereof include organic acid salts, inorganic acid salts, organic bases, and inorganic bases. Examples of the organic acid salts include monocarboxylates such as acetate, trifluoroacetate, butyrate, palmitate, and stearate; polyhydric carboxylates such as fumarate, maleate, succinate, and malonate; oxycarboxylates such as lactate, tartrate, and citrate; and organic sulfonates such as methanesulfonate and toluenesulfonate such as tosylate. Examples of the inorganic acid salts include hydrochloride, sulfate, nitrate, hydrobromide, and phosphate. Examples of the salt with an organic base include a salt with an organic amine such as methylamine, triethylamine, triethanolamine, diethanolamine, morpholine, piperazine, pyrrolidine, terpyridine, picoline, and ethylenediamine. Examples of the salt with an inorganic base include various salts such as an ammonium salt; and a salt with a metal such as an alkali metal such as sodium and potassium, an alkaline earth metal such as calcium and magnesium, and aluminum. Each one of these salts may be used alone, or any combination of two or more of them may be used. The above salts may include solvates or hydrates of the salts.

The above Compound (1) may also be obtained by known methods such as synthesis methods, extraction methods, and fermentation methods.

As an example of the synthesis method of the above Compound (1), L-ergothioneine may be subjected to an appropriate oxidation treatment to obtain said compound. As an example of the above synthesis method, L-ergothioneine may be heat-treated at a high temperature or for an extended period of time, and then subjected to preparative separation, purification, or the like by high-performance liquid chromatography or the like to obtain said compound.

As a more specific example, an aqueous solution of L-ergothioneine may be subjected to a basic condition or heat-treatment to synthesize said compound, and it is preferable to use a method including the step (1) for heat-treating it at 60°C or more for 24 hours or more. Furthermore, it is more preferable to use a method including the step (1) for heat-treating it at 60°C or more at pH 9-11 for 24 hours or more. Also, the above step (1) may be carried out at, for example, 80°C or more to carry out, for example, a solution synthesis in which L-ergothioneine is used at a concentration of 1500 mM or the like as an aqueous solution. Also, the concentration of the aqueous solution of L-ergothioneine is, for example, preferably 1 mM or more, more preferably 10 mM or more, and still more preferably 100 mM or more, depending on the intended use or usage. Also, the concentration of the aqueous solution of L-ergothioneine may be, for example, 2 M or less, 1.8 M or less, 1.5 M or less, 1.3 M or less, or the like, depending on the intended use or usage.

### [Composition]

The composition of the present invention contains the above Compound (1). The Compound (1) may be contained alone, or in two or more kinds thereof.

In the composition of the present invention, the content of the above Compound (1) is appropriately adjusted depending on the usage of the composition, the types and contents of other ingredients, and the like, and not specifically limited. For example, the content may be 0.000001% by mass or more, and examples thereof include 0.0000015% by mass or more, 0.000005% by mass or more, 0.00001% by mass or more, 0.00005% by mass or more, 0.0001% by mass or more, 0.0005% by mass or more, and 0.001% by mass or more, relative to the total mass of the composition. Also, the content of the above Compound (1) may be, for example, 99.999% by mass or less, or 99.9% by mass or less, 99.5% by mass or less, 99% by mass or less, 98.5% by mass or less, 98% by mass or less, or 80% by mass or less, and examples thereof include 70% by mass or less, 60% by mass or less, 50% by mass or less, 40% by mass or less, 30% by mass or less, 20% by mass or less, 10% by mass or less, 5% by mass or less, and 1% by mass or less, relative to the total mass of the composition. In another embodiment, for example, when the Compound (1) is prepared as a liquid composition or a liquid formulation, the content of the Compound (1) is not specifically limited, but may be, for example, 80% by mass or less, and examples thereof include 70% by mass or less, 60% by mass or less, 50% by mass or less, 40% by mass or less, 30% by mass or less, 20% by mass or less, 10% by mass or less, 5% by mass or less, and 1% by mass or less, relative to the total mass of the composition.

Also, the above composition may further contain known various ingredients and additives depending on the usage of the composition and the types of other ingredients. Examples of the above ingredients and additives include excipients, lubricants, binders, and disintegrants; and for liquid formulations, examples thereof include solvents, solubilizing agents, emulsifiers, emulsion stabilizers, thickeners, humectants, suspending agents, tonicity agents, buffering agents, soothing agents, preservatives, antioxidants, colorants, sweetening agents, and perfumes. They may be used each alone, or in a mixture of two or more kinds of them.

### [Hydrogen peroxide remover]

The hydrogen peroxide remover of the present invention contains the above Compound (1). The Compound (1) may be contained alone, or in two or more kinds thereof.

The hydrogen peroxide remover of the present invention can achieve, for example, effects for appropriately removing hydrogen peroxide by using the above constitution.

In the hydrogen peroxide remover of the present invention, the content of the above Compound (1) (the total content in case two or more kinds thereof exist) is appropriately adjusted depending on the usage of the hydrogen peroxide remover, the types and contents of other ingredients, and the like, and not specifically limited. For example, the content may be 0.000001% by mass or more, and examples thereof include 0.0000015% by mass or more, 0.000005% by mass or more, 0.00001% by mass or more, 0.00005% by mass or more, 0.0001% by mass or more, 0.0005% by mass or more, and 0.001% by mass or more, relative to the total mass of the hydrogen peroxide remover. Also, the content of the above compound may be, for example, 99.999% by mass or less, or 99.9% by mass or less, 99.5% by mass or less, 99% by mass or less, 98.5% by mass or less, 98% by mass or less, or 80% by mass or less, and examples thereof include 70% by mass or less, 60% by mass or less, 50% by mass or less, 40% by mass or less, 30% by mass or less, 20% by mass or less, 10% by mass or less, 5% by mass or less, and 1% by mass or less, relative to the total mass of the hydrogen peroxide remover. In another embodiment, for example, when the Compound (1) is prepared as a liquid hydrogen peroxide remover or a liquid formulation, the content of the Compound (1) is not specifically limited, but may be, for example, 80% by mass or less, and examples thereof include 70% by mass or less, 60% by mass or less, 50% by mass or less, 40% by mass or less, 30% by mass or less, 20% by mass or less, 10% by mass or less, 5% by mass or less, and 1% by mass or less, relative to the total mass of the hydrogen peroxide remover.

When the above hydrogen peroxide remover is used in a liquid form, the concentration of the above Compound (1) may be used at a concentration of, for example, 0.01 µM to 0.1 µM, 0.02 µM to 0.08 µM, 0.03 µM to 0.06 µM, 0.04 µM to 0.05 µM, or the like, depending on the intended use or usage. Also, for example, preferable examples thereof include a case wherein the concentration of the above Compound (1) is used at a concentration of 0.04 µM or more.

Also, the above hydrogen peroxide remover may further contain the above known various ingredients and additives depending on the usage of the hydrogen peroxide remover and the types of other ingredients. They may be used each alone, or in a mixture of two or more kinds of them.

### [Cytotoxicity inhibitor]

The cytotoxicity inhibitor of the present invention contains the above Compound (1), and is used in the presence of hydrogen peroxide. The Compound (1) may be contained alone, or in two or more kinds thereof.

The cytotoxicity inhibitor of the present invention can achieve, for example, effects for reducing the cytotoxicity caused by hydrogen peroxide in a medium by using the above constitution.

In the cytotoxicity inhibitor of the present invention, the content of the above Compound (1) (the total content in case two or more kinds thereof exist) is appropriately adjusted depending on the usage of the cytotoxicity inhibitor, the types and contents of other ingredients, and the like, and not specifically limited. For example, the content may be 0.000001% by mass or more, and examples thereof include 0.000005% by mass or more, 0.00001% by mass or more, 0.00005% by mass or more, 0.0001% by mass or more, 0.0005% by mass or more, and 0.001% by mass or more, relative to the total mass of the cytotoxicity inhibitor. Also, the content of the above Compound (1) may be, for example, 99.999% by mass or less, or 99.9% by mass or less, 99.5% by mass or less, 99% by mass or less, 98.5% by mass or less, 98% by mass or less, or 80% by mass or less, and examples thereof include 70% by mass or less, 60% by mass or less, 50% by mass or less, 40% by mass or less, 30% by mass or less, 20% by mass or less, 10% by mass or less, 5% by mass or less, and 1% by mass or less, relative to the total mass of the cytotoxicity inhibitor. In another embodiment, for example, when the Compound (1) is prepared as a liquid cytotoxicity inhibitor or a liquid formulation, the content of the Compound (1)is not specifically limited, but may be, for example, 80% by mass or less, and examples thereof include 70% by mass or less, 60% by mass or less, 50% by mass or less, 40% by mass or less, 30% by mass or less, 20% by mass or less, 10% by mass or less, 5% by mass or less, and 1% by mass or less, relative to the total mass of the cytotoxicity inhibitor.

When the above cytotoxicity inhibitor is used in a liquid form, the concentration of the above compound or a salt thereof may be used at a concentration of, for example, 0.01 µM to 10 µM, 0.1 µM to 5 µM, 1 µM to 3 µM, or the like, depending on the intended use or usage.

Also, the above cytotoxicity inhibitor may further contain the above known various ingredients and additives depending on the usage of the cytotoxicity inhibitor and the types of other ingredients. They may be used each alone, or in a mixture of two or more kinds of them.

### EXAMPLES

Next, the present invention is specifically illustrated by way of Examples, but the present invention is not limited to the following Examples.

### [Example 1]

### (Preparation of compound)

A compound represented by the following formula (1) (hereinafter also referred to as "Compound A" or "EGT-X") was prepared according to the following method.

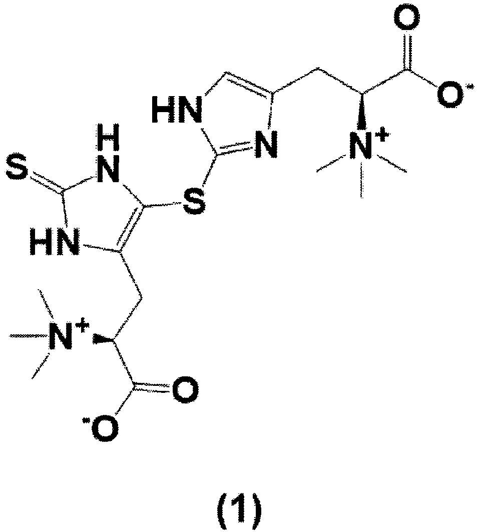

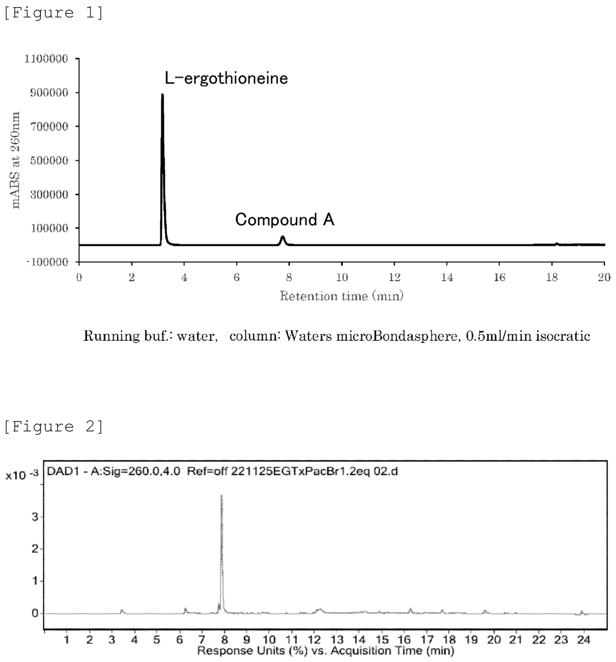

L-Ergothioneine (manufactured by Tetrahedron) was dissolved in N-cyclohexyl-3-aminopropanesulfonic acid buffer (pH 10) to prepare a 250 mM ergothioneine solution (20 mL). After a heat-treatment at 60°C for 96h, the solution was subjected to an HPLC analysis under the following conditions (Figure 1).
Device: Shimadzu LC20A (manufactured by Shimadzu Corporation)
Column: µBondasphere C18 5 µm 100 A, 150 x 3.9 mm I.D. (manufactured by Waters Corporation)
Flow rate: 0.5 mL/min, Temperature: 30°C
Detector: PDA (260 nm)
Eluent: Distilled water isocratic
Retention time: about 3.3 min. (L-ergothioneine), about 7.8 min. (Compound A)
Device: Medium pressure column preparative system EPCLC AI-580S (manufactured by YAMAZEN)
Column: ODS-C-50C 37 X 300 mm (manufactured by YAMAZEN)
Flow rate: 6 mL/min, Temperature: Room temperature
Detector: UV (260 nm)
Eluent: Distilled water isocratic
Retention time: about 20 min. (L-ergothioneine), about 30 min. (Compound A)
Injection amount: 0.5-0.6 mL

Preparative separations were carried out ten times under the above conditions, and the resulting fractions containing the Compound A were collected.

The resulting fraction solution was concentrated from about 1000 mL to 30 mL using a rotary evaporator (in hot-water bath at 60°C). Subsequently, the resulting solution was lyophilized to obtain the Compound A (108 mg) as yellow powder. The resulting Compound A was redissolved in D₂O to carry out a NMR analysis.

### (¹H-NMR measurement)

Result of ¹H-NMR measurement is shown below. ¹H-NMR (400 MHz, D₂O) DSS-d₆ was as 0 ppm.
δ 7.00 (s,1H), 3.91 (m, 2H), 3.33 (m, 2H), 3.29 (s, 9H), 3.24 (s, 9H), 3.14(m, 2H)

### (¹³C-NMR measurement)

Result of ¹³C-NMR measurement is shown below. ¹³C-NMR (400 MHz, D₂O) DSS-d₆ was set as 0 ppm.
δ 173.49, 172.93, 169.98, 140.47, 136.72, 134.11, 122.56, 117.67, 80.98, 79.37, 54.79, 28.00, 25.65

### (High resolution mass spectrometry)

Result of liquid chromatography-high resolution mass spectrometry (LC-HRMS) is shown below.
(LC-HRMS analysis conditions)
Device: Agilent Technologies 6224 TOFLC/MS (manufactured by Agilent Technologies, Inc.)
Column: µBondasphere C18 5 µm 100 A, 150 x 3.9 mm I.D. (manufactured by Waters Corporation)
Flow rate: 0.5 mL/min, Temperature: 30°C
Eluent: Distilled water isocratic
Ionization condition: ESI, Capillary voltage 3500 V, Fragmenter voltage 100 V
Retention time: about 7.8 min. (Compound A)

The theoretical value of [M+H]⁺ of C₁₈H₂₈N₆O₄S₂ was 457.1686, while the actual value was 457.1686.

Also, in order to confirm whether the Compound A has two or more thione groups, a reaction test with an S-modification reagent phenacyl bromide was carried out.

An aqueous solution (100 µL) of the Compound A (32 mg/mL) and a solution (100 µL) of phenacyl bromide (manufactured by Tokyo Chemical Industry Co., Ltd.) (15 mg/mL) in N,N-dimethylformamide was mixed, and the resulting mixture was incubated at room temperature for 10 minutes. The mixture was subjected to liquid chromatography-high resolution mass spectrometry (LC-HRMS) under the following conditions.
Device: Agilent Technologies 6224 TOFLC/MS (manufactured by Agilent Technologies, Inc.)
Column: µBondasphere C18 5 µm 100 A, 150 x 3.9 mm I.D. (manufactured by Waters Corporation)
Flow rate: 0.5 mL/min, Temperature: 30°C
Detector: PDA (260nm)
Eluent:0.1% formic acid (A), acetonitrile (B) gradient
Gradient condition: 0% B (0 min.) → 100% B (20 min.)
Ionization condition: ESI, Capillary voltage 3500 V,
Fragmenter voltage 100 V

The result is shown in Figure 2. The detected major peak was one at the retention time of about 7.9 min.

The result of liquid chromatography-high resolution mass spectrometry (LC-HRMS) of the major peak at the retention time of about 7.9 min. is shown below.

The theoretical value of [M+H]⁺ of C₂₆H₃₄N₆O₅S₂ was 575.2105, while the actual value was 575.2099, which confirms that the obtained compound was one to which one phenacyl group was bound (Figure 2).

### [Example 2]

### (Evaluation of hydrogen peroxide removal)

The resulting Compound A (EGT-X) was subjected to several doubling dilutions from 10 µM with the buffer provided with a kit (OxiSelect hydrogen peroxide assay kit (manufactured by CELL BIOLABS, Inc.)), and 25 µL each of the resulting solutions was added to a 96 well plate. Also, a 4 µM solution in which hydrogen peroxide was diluted with the buffer provided with the kit was prepared, and 25 µL each of said solution was added to the 96 well plate. Further, a blank was prepared under the same conditions except for no hydrogen peroxide was added.

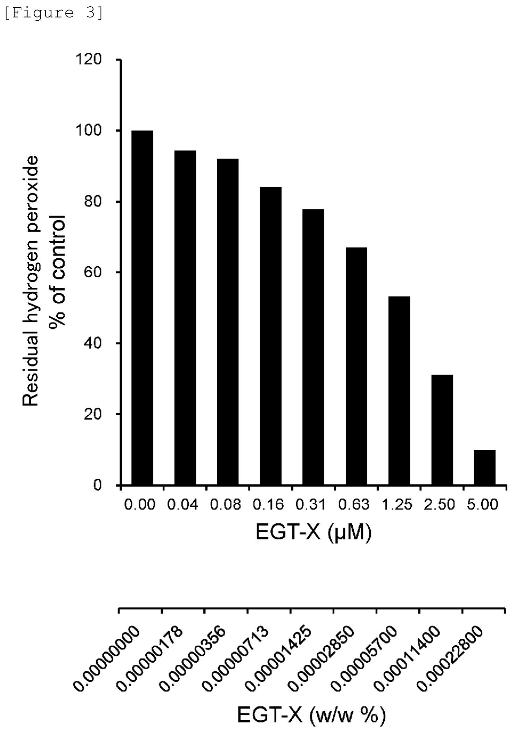

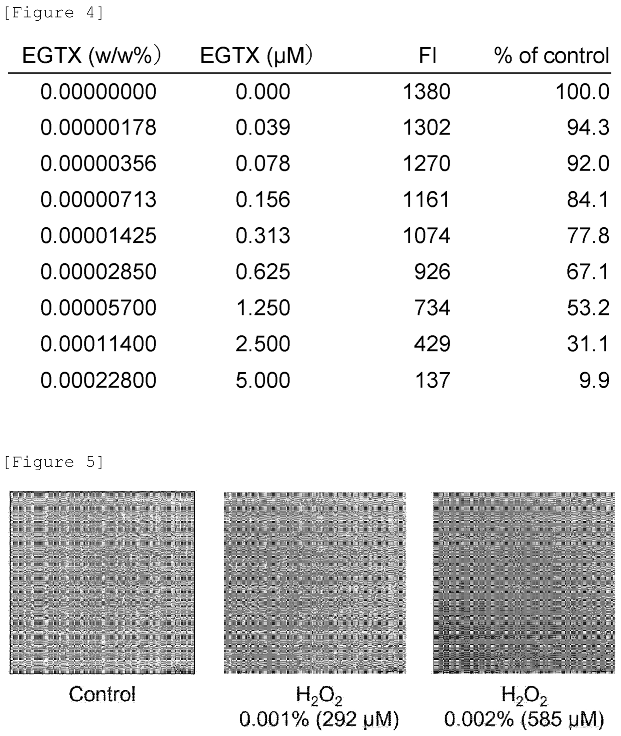

Subsequently, according to the protocol described in the kit, 50 µL each of a mixture of ADHP (10-acetyl-3,7-dihydroxyphenoxazine) and HRP(horseradish peroxidase) provided with the kit was added thereto, and the resulting mixture was stirred. After incubated at room temperature for 30 minutes, the fluorescence intensity was measured under the condition of excitation wavelength of 530 nm and fluorescence wavelength of 590 nm. The blank value was subtracted from each measurement value to calculate each value. The percentage values when EGT-X was added were calculated based on the control (Compound A: 0 µM) value set as 100%, and the results were plotted in a graph. (Figures 3 and 4).

As shown in Figures 3 and 4, as the amount of the Compound A (EGT-X) increased, the amount of the residual hydrogen peroxide decreased.

### [Example 3]

### (Evaluation of cytotoxicity inhibition)

For the evaluation of cytotoxicity inhibition, hydrogen peroxide cytotoxicity against HEK293 cells was evaluated.

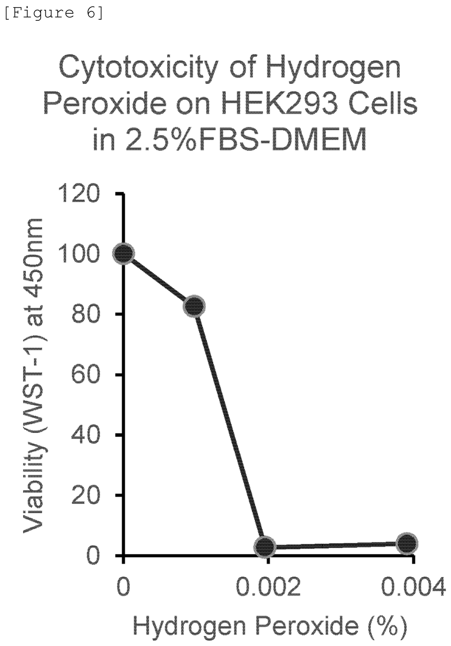

A 100 µL each of a suspension of 15 x 10⁴ cells/mL of HEK293 cells (Dulbecco's modified Eagle's medium (DMEM) containing 5% fetal bovine serum (FBS)) was added to a 96 well plate. After 24 hours, 100 µL each of a DMEM only or a DMEM containing hydrogen peroxide was added thereto so that the hydrogen peroxide concentration would be 0, 0.001, 0.002, or 0.004%. The cell viability when exposed to hydrogen peroxide for 24 hours was observed. In order to measure the cell viability, 50 µL of a 1:1 mixture of a DMEM containing 5% FBS and WST-1 Premix (manufactured by Takara Bio Inc.) was added thereto, and the resulting mixture was incubated in a 5% CO₂ incubator for 30 minutes. The absorbance at 450 nm was measured. A blank value was obtained under the same conditions as the above conditions except for adding no cells, and the blank value was subtracted from the above result to calculate the cell viability (Figures 5 and 6).

A 100 µL each of a suspension of 15 x 10⁴ cells/mL of HEK293 cells (5% FBS-DMEM) was added to a 96 well plate. After 24 hours, 50 µL each of EGT-X dissolved in a DMEM and hydrogen peroxide dissolved in a DMEM was added to the plate, so that the hydrogen peroxide concentration would be 0 for the control and 0.0015% for the others, and the EGT-X concentration would be those shown in Figures (Figures 5 and 6). Each mixture was cultured for additional 24 hours, and in order to measure the cell viability, 50 µL of a 1:1 mixture of a DMEM containing 5% FBS and WST-1 Premix (manufactured by TAKARA Bio Inc.) was added thereto, and the resulting mixture was incubated in a 5% CO₂ incubator for 30 minutes. The absorbance at 450 nm was measured. A blank value was obtained under the same conditions as the above conditions except for adding no cells, and the blank value was subtracted from the above result to calculate the cell viability.

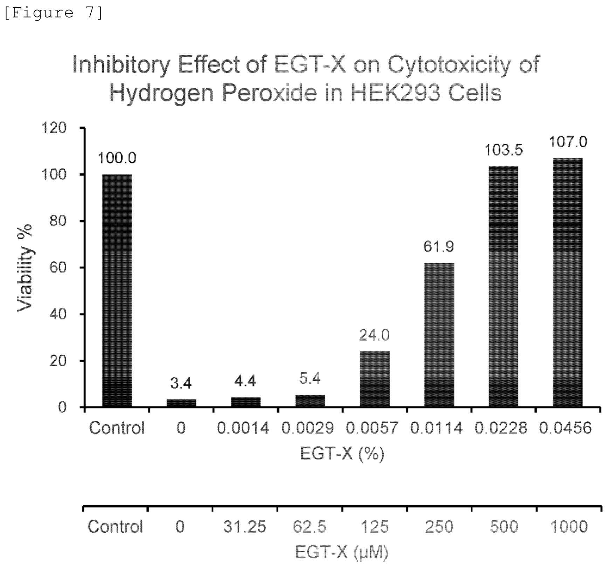

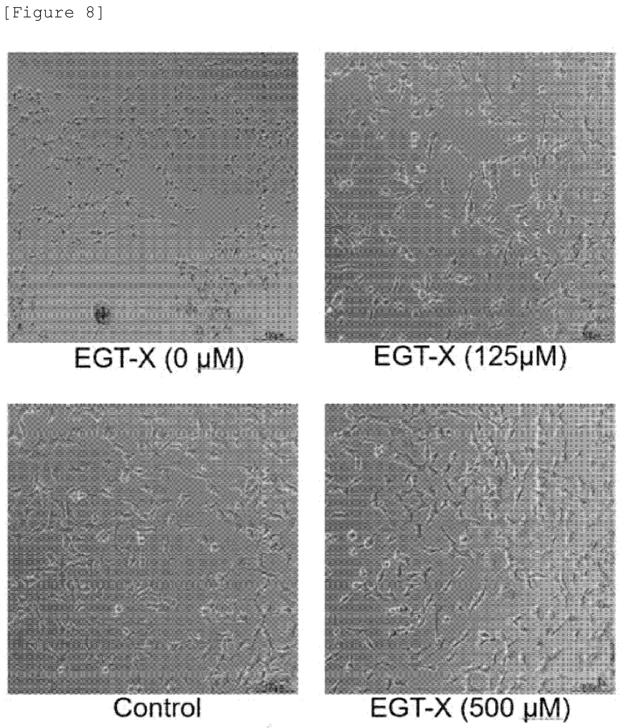

The graph shows the results when cell viability without hydrogen peroxide and EGT-X (control) was set as 100% (Figures 7 and 8).

As shown in Figures 5 and 6, only a slight decrease in viability was observed at 0.001% (292 µM) of hydrogen peroxide, and cells were killed at 0.002% (585 µM). Therefore, the effects of EGT-X on the cytotoxicity caused by hydrogen peroxide at the intermediate value, i.e., the hydrogen peroxide concentration of 0.0015%, were determined to be investigated.

Also, as shown in Figures 7 and 8, the cytotoxicity caused by hydrogen peroxide was inhibited in the presence of 125 µM or more of EGT-X.

## Claims

1. A compound represented by the following formula (1) or a salt thereof

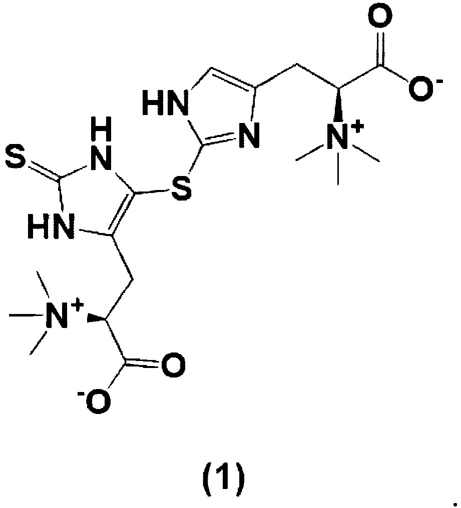

2. A composition comprising the compound according to claim 1 or a salt thereof.

3. A hydrogen peroxide remover comprising the compound according to claim 1 or a salt thereof.

4. The hydrogen peroxide remover according to claim 3, wherein a concentration of said compound or a salt thereof is used at a concentration of 0.04 µM or more.

5. A cytotoxicity inhibitor comprising the compound according to claim 1 or a salt thereof, which is used in the presence of hydrogen peroxide.

6. A method for producing a compound represented by the following formula (1) or a salt thereof, which comprises a step (1) for heat-treating an aqueous solution of L-ergothioneine at 60°C or more for 24 hours or more.
